Europäisches Patentamt

European Patent Office

Office européen des brevets

(11) Publication number: 0 297 456
A2

# EUROPEAN PATENT APPLICATION

(21) Application number: 88110111.7

(22) Date of filing: 24.06.88

(51) Int. Cl.⁴: **C07K 7/08 , C07K 7/06 , A61K 37/02 , C07K 1/04**

(30) Priority: 29.06.87 US 67839

(43) Date of publication of application:
04.01.89 Bulletin 89/01

(84) Designated Contracting States:
AT BE CH DE ES FR GB GR IT LI LU NL SE

(71) Applicant: MERRELL DOW
PHARMACEUTICALS INC.
2110 East Galbraith Road
Cincinnati Ohio 45215-6300(US)

(72) Inventor: Cardin, Alan D.
9903 Hunters Run Court
Cincinnati Ohio 45242(US)
Inventor: Bowlin, Terry L.
8466 Pond Ridge Drive
Maineville Ohio 45039(US)
Inventor: Krstenansky, John L.
3749 Ault Park
Cincinnati Ohio 45208(US)

(74) Representative: Macchetta, Francesco
Gruppo Lepetit S.p.A. Patent and Trademark
Department 34, Via Roberto Lepetit
I-21040 Gerenzano (Varese)(IT)

(54) Immunosuppressant peptides.

(57) Novel peptides having a fragment containing 2 or 3 Arg or Lys residues and having a fragment containing 2 or 3 Gly, Leu, Ile, or Thr residues adjacent to the carboxy end of the first fragment and having amino and carboxy tails consisting of chains made up of any amino acids are immunosuppressant agents and can suppress the lymphocyte system by suppressing lymphocyte proliferation.

## IMMUNOSUPPRESSANT PEPTIDES

This invention relates to novel peptides and their immunosuppresant properties.

The immune system is one of the primary defenses against disease bearing microbes and other foreign antigens in higher animals. An immune response is mediated by the action of specific immune cells which react to specific antigens. Potential antigens can be a variety of substances, often proteins, which are foreign to an individual's body. They are most frequently located on the outer surfaces of cells. Potential antigens can be found on pollen grains, tissue grafts, animal parasites, viruses, and bacteria.

In humans, many potential antigens never pass the body's first two defense lines and therefore never trigger the immune system. These two defense lines consist firstly of the skin, mucous membranes, tears, and stomach acid and secondly of specialized white blood cells, granulocytes and monocytes, and macrophages which destroy pathogens and other potential antigens by phagocytosis, that is, by engulfing and destroying the foreign material. These white blood cells and macrophages are called phagocytes. When pathogens or other foreign substances do pass the body's first two defense lines, the immune response begins.

There are two principal immune defense systems, humoral and cellular, both of which react to antigens. Humoral immunity is due to circulating antibodies which are found in the gamma globulin fraction of the plasma proteins. When plasma is centrifuged at high speeds its component proteins separate by weight into sections called fractions. Antibodies are usually found in the fraction whose components have a molecular weight of approximately 156,000. This particular fraction has been named the gamma globulin fraction. Humoral immunity forms a major defense against bacterial infections. Cellular immunity is partly due to lymphocyte products called lymphokines. This type of immunity is responsible for delayed allergic reactions, rejection of transplants of foreign tissue, and rejection of tumor cells. It is the major defense against infections due to viruses, fungi, and a few bacteria such as the tubercle bacillus.

Specialized white blood cells called lymphocytes are responsible for both humoral and cellular immunity. Lymphocyte precursor cells are made in the bone marrow of adult humans followed by migration to various organs or in the yolk sac of a developing fetus followed by migration into the fetus and then to various organs. In humans, some of these precursor cells migrate to the thymus, which is a two-lobed, glandular appearing structure located in the upper chest just behind the sternum, where they are transformed into T-lymphocytes, which are involved in cellular immunity. In humans, the rest of the precursor cells migrate to the spleen where they are transformed into B-lymphocytes, which are involved in humoral immunity. The T- and B-lymphocytes are structurally indistinguishable although they function differently and can be distinguished through various chemical means. The mature lymphocytes circulate in the blood and can also be found in the lymph nodes as well as the spleen and thymus.

Humoral immunity is mediated by the B-lymphocytes which have receptors for particular antigens on their cell surfaces. They seem to be very specific and each type of B-lymphocyte reacts to only one antigen. When bacteria or viruses, for example, invade an organism, B-lymphocytes react to and combine with the antigens on the bacterial or viral surface and the lymphocyte is stimulated to divide. Its daughter cells are transformed into specialized cells called plasma cells. These cells produce and then secrete large quantities of antibodies into the general circulation. The antibodies are specific for the antigens which stimulated their production and react only with those antigens. Antibodies known as agglutinins cause several antigen containing substances to agglutinate or clump together. This keeps the substance from spreading to the tissues and allows the phagocytes to capture or the lymph nodes to filter the invading material. Other antibodies act by opening holes in bacterial cell walls, thereby killing the bacteria. These are known as lysins. Antibodies call antitoxins combine with toxins produced by bacteria and thereby neutralize them.

Once a pathogen invades the body and the immune response begins, antibodies can be made in several hours. This initial reaction is called the primary response or primary immunization. However, during that time, the pathogens have also been dividing and sometimes producing toxin, either of which results in various disease symptoms. It may take days or weeks before enough antibodies are made to eliminate all the pathogens but once they disappear, the disease symptoms disappear as well. The lymphocytes, plasma cells, and antibodies remain and circulate in the blood so that if the same pathogens enter the body a second time, the lymphocytes react immediately and start antibody production. The response of the sensitized lymphocytes is called the secondary response. The secondary response results in the production of higher levels of antibody than were produced during the primary response. So many antibodies are produced so rapidly that the microbes are unable to divide and cause disease. This type of humoral immunity is known as immediate hypersensitivity due to the fact that a previously exposed organism can respond within minutes to an antigen, as in the case of hay fever. Another example of immediate hypersensitivity would be anaphylactic shock, an extreme allergic reaction that sometimes occurs when an individual is exposed to an antigen to which he has been sensitized. At times, this humoral response to the antigen can result in death.

Humoral immunity can be both naturally and artificially induced. In the case of active natural immunity, an individual's lymphocytes continue to circulate and activate the production of antibodies after an infection. This active natural immunity lasts for many years or even a lifetime. An infant receives antibodies from the colostrum, milk secreted by the mother, the first few days after birth, which gives it immunity the first year of its life. This is known as passive natural immunity since the infant is not involved in the actual production of the antibodies. Active artificial immunity is induced by injecting dead or weakened microbes into an individual. Their surface antigens can still trigger lymphocyte production of antibodies but these microbes do not cause the disease symptoms that their more virulent forms do not cause the disease symptoms that their more virulent forms do. When the individual is later exposed to the virulent microbe, he is already sensitized and immediately responds with a massive production of antibodies. Active artificial immunity may last many years or permanently with booster shots. There is also a form of passive artificial immunity which provides protection for about one month. This temporary immunity is brought about by injecting antibodies obtained from another person or animal into an individual. It is usually only used in crisis situations and epidemics. Because the lymphocytes are bypassed, they neither make antibodies nor "remember" the antigen, which accounts for the temporary effect of this method.

In cellular immunity, as contrasted to humoral-immunity, circulating antibodies are not detectable. The T-lymphocytes which mediate this type of immunity are activated when they encounter antigens on cells from another individual, as in the case of transplants, tumors, or viruses. Like B-lymphocytes, T-lymphocytes are specific and each type reacts with only one antigen. The lymphocytes enlarge, divide, and produce lymphokines which participate in the attack on the foreign antigen. They also stimulate the phagocytic activity of macrophages. Although immunological memory exists as with humoral immunity, the response is much slower. It may take as long as ten or twelve hours to develop a response in a previously sensitized individual and cellular immunity is therefore known as delayed hypersensitivity. The allergic reaction to poison ivy, oak, and sumac, the red splotch seen in a positive tuberculin skin test, and rejection of transplant tissue are all cellular immune responses.

Immunomodulating agents activate or inhibit the process of lymphocyte proliferation. Normal lymphocyte proliferation is due to various interactions between antigens, macrophages, T- and B-lymphocytes as well as certain chemicals. For example, the presence of a particular antigen activates a particular T- or B-lymphocyte. Additionally, certain B-lymphocytes can be activated by active T-lymphocytes while others are independent of the T-lymphocytes and are activated only by antigens. Activated T-lymphocytes can cause macrophages to produce a molecule known as interleukin 1(IL-1) which in turn activates both T- and B-lymphocytes. Activated T-lymphocytes can also produce a molecule known as interleukin 2(IL-2) which further induces T-lymphocyte activation. Chemicals, called mitogens can trigger DNA synthesis and mitosis, which are signs of activity in T-or B-lymphocytes. Some mitogens affect only one type of lymphocyte while others affect may types. Immunomodulating agents of various kinds and in varying amounts affect the complex interactions between the components of the immune system. As will be shown, the compounds and compositions of this invention act as immune suppressors and affect both T- and B-lymphocytes.

Although the immune system is a major defense against substances which can cause disease, it cannot distinguish between helpful and harmful foreign substances and destroys both. It would be useful in many instances to have a means of regulating the immune system without harming the individual. The peptides of this invention exhibit such modulating or regulatory effects and have potential for use in the treatment of various immune disorders.

There are times when the individual's immunological response causes more damage or discomfort than the invading microbes or foreign material, as in the case of allergic reactions. Suppression of the immune response in these cases would be desirable.

Occasionally, the immunological mechanisms become sensitized to some part of the individual's own body causing interference with or even destruction of that part. The ability to distinguish between "self" and "not self" is impaired and the body begins to destroy itself. Some examples of these autoimmune diseases in man are rheumatoid arthritis, certain hemolytic anemias, rheumatic fever, thyroiditis, ulcerative colitis, myasthenia gravis, glomerulonephritis - a kidney disease, allergic encephalomyelitis, continuing nerve and liver destruction which sometimes follows viral hepatitis, and possibly multiple sclerosis and systemic lupus erythematosus. Some forms of autoimmunity come about as the result of trauma to an area usually not exposed to lymphocytes such as neural tissue or the lens of the eye. When the tissues in these areas becomes exposed to lymphocytes, their surface proteins can act as antigens and trigger the production of antibodies and cellular immune responses which then begin to destroy those tissues. Other auto-immune diseases develop after exposure of the individual to antigens which are antigenically similar to, that is cross-react with, the individual's own tissue. Rheumatic fever is an example of this type of disease in which the antigen of the streptococcal bacterium which causes rheumatic fever is cross-reactive with parts of the human heart. The antibodies cannot differentiate between the bacterial antigens and the heart muscle antigens and cells with either of those antigens can be destroyed. Suppression of the immune system in these autoimmune diseases could be useful in minimizing or eliminating the effects of the disease.

Circulating antibodies and cellular immune responses play a role in the rejection of transplanted tissues and organs. Unless the donor is the identical twin of the recipient or is the individual himself, the recipient's lymphocytes recognize the transplant as "not self" and immediately respond to destroy it. The exceptions to this situation are transplants to non-vascularized areas (privileged sites), such as the cornea of the eye, where lymphocytes do not circulate and therefore are not sensitized and do not prompt an immune response. It is currently difficult to suppress the immune reaction to prevent rejection of the transplant without severly damaging the patient in other ways. The patient must also be given massive doses of antibiotics because his own defenses against infection have been suppressed. The compounds and compositions of this invention could be valuable in establishing tolerance to the transplant through controlled modulation of the immune system.

Compounds such as those disclosed in this invention, which suppress the immune system are of importance. They are particularly useful when the normal action of the system has been disturbed or has caused the destruction of harmless foreign materials such as transplanted organs.

## SUMMARY OF THE INVENTION

Peptides of Structure 1:

Tn-A-B-C-D-Tc     (1)

wherein

Tn is an amino terminal residue selected from hydrogen, one or two alkyl groups of from one to six carbon atoms, one or two acyl groups of from 2 to 10 carbon atoms, carbobenzyloxy, or t-butyloxycarbonyl;

A is a peptide fragment containing from 4 to 12 amino acid residues selected from any amino acid;

B is a peptide fragment containing 2 or 3 amino acid residues selected from Arg and Lys;

C is a peptide fragment containing 2 or 3 amino acid residues selected from Gly, Leu, Ile, and Thr;

D is a peptide fragment containing from 1 to 12 amino acid residues selected from any amino acid;

Tc is a carboxy terminal residue selected from OH, $(C_1-C_6)$alkoxy, amino, mono- or di-$(C_1-C_4)$alkyl substituted amino, or benzylamino
are useful immunosuppresant agents.

## DETAILED DESCRIPTION OF THE INVENTION

The following common abbreviations of the naturally occurring amino acid are used throughout this specification:

Gly - glycine
Ala - alanine
Val - valine
Leu - leucine
Ile - isoleucine
Pro - proline
Phe - phenylalanine
Trp - tryptophan
Met - methionine
Ser - serine
Thr - threonine
Cys - cysteine
Tyr - tyrosine
Asn - asparagine

4

Gln - glutamine
Asp - aspartic acid
Glu - glutamic acid
Lys - lysine
Arg - arginine
His - histidine
Nle - norleucine

The natural amino acids, with the exception of glycine, contain a chiral carbon atom. Unless otherwise specifically indicated, the optically active amino acids, referred to herein, are of the L-configuration. As is customary, the structure of peptides written out herein is such that the amino terminal end is on the left side of the chain and the carboxy terminal end is on the right side of the chain.

An alkyl group or the alkyl portion of an alkoxy group is taken to include straight, branched, or cyclic alkyl groups, for example, methyl, ethyl, propyl, isopropyl, butyl, isobutyl, pentyl, isopentyl, sec-pentyl, cyclopentyl hexyl, isohexyl, cyclohexyl, and cyclopentylmethyl.

An acyl group of from 2 to 10 carbon atoms is taken to include straight, branched, and cyclic, saturated and unsaturated acyl groups having one or two carbonyl moieties, for example, acetyl, succinyl, or benzoyl.

The term "any amino acid" as used herein includes the naturally occurring amino acids, as well as other "non-protein" α-amino acids commonly utilized by those in the peptide chemistry arts when preparing synthetic analogs of naturally occurring polypeptides.

The naturally occurring amino acids are glycine, alanine, valine, leucine, isoleucine, serine, methionine, threonine, phenylalanine, tyrosine, tryptophan, cysteine, proline, histidine, aspartic acid, asparagine, glutamic acid, glutamine, arginine, ornithine, and lysine. Examples of "non-protein" α-amino acids are norleucine, norvaline, alloisoleucine, homoarginine, thiaproline, dehydroproline, hydroxyproline (Hyp), homoserine, cyclohexylglycine (Chg), α-amino-n-butyric acid (Aba), cyclohexylalalnine (Cha), aminophenylbutyric acid (Pba), phenylalanines substituted on the phenyl moiety with alkyl, alkoxy, halogen, or nitro groups, O-alkylated derivatives of serine, threonine, or tyrosine, S-alkylated cysteine, O-sulfate ester of tyrosine, and the D-isomers of the naturally occurring amino acids.

As is true for any group of agents having pharmaceutical activity, certain definitions of the various groups are preferred. Here, peptides having Tn being hydrogen and Tc being OH or amino are preferred. Also preferred are those peptides wherein A is a peptide fragment containing from 3 to 6 amino acid residues selected from the natural amino acids. Also preferred are those peptides wherein D is a peptide fragment containing 1 to 12 amino acid residues selected from any natural amino acid. More preferred are those peptides wherein C is a peptide fragment containing 2 or 3 amino acid residues selected from Gly, Leu, Ile and Thr.

The polypeptides of formula (1) can form pharmaceutically acceptable salts with any non-toxic, organic or inorganic acid. Illustrative inorganic acids which form suitable salts include hydrochloric, hydrobromic, sulphuric and phosphoric acid and acid metal salts such as sodium monohydrogen orthophosphate and potassium hydrogen sulfate. Illustrative organic acids which form suitable salts include the mono, di- and tricarboxylic acids. Illustrative of such acids are, for example, acetic, glycolic, lactic, pyruvic, malonic, succinic, glutaric, fumaric, malic, tartaric, citric, ascorbic, maleic hydroxymaleic, benzoic, hydroxybenzoic, phenylacetic, cinnamic, salicylic, 2-phenoxybenzoic and sulfonic acids such as methane sulfonic acid and 2-hydroxyethane sulfonic acid. Salts of the carboxy terminal amino acid moiety include the non-toxic carboxylic acid salts formed with any suitable inorganic or organic bases. Illustratively, these salts include those of alkali metals, as for example, sodium and potassium; alkaline earth metals, such as calcium and magnesium; light metals of Group IIIA including aluminum; and organic primary, secondary and tertiary amines, as for example, trialkyl-amines, including triethylamine, procaine, dibenzylamine, 1-ethenamine, N,N′-dibenzyl-ethylenediamine, dihydroabietylamine, (N-(lower)alkyl-piperidine, and any other suitable amine.

The following peptides are illustrative of those of this invention:

H-Thr-Arg-Leu-Thr-Arg-Lys-Arg-Gly-Leu-Lys-Leu-Ala-Thr-Ala-Leu-NH$_2$;
H-Leu-Arg-Lys-Leu-Arg-Lys-Arg-Leu-Leu-Arg-Asp-Ala-Asp-Asp-Leu-NH$_2$;
H-Leu-Gln-Asn-Arg-Arg-Gly-Leu-Asp-Leu-Leu-Phe-Leu-Lys-Glu-Gly-Gly-Leu-NH$_2$; and
H-Glu-Val-Val-Leu-Gln-Asn-Arg-Arg-Gly-Leu-Leu-OH.

The proteins of this invention can be prepared by a variety of procedures readily known to those skilled in the art. Such procedures include the solid phase sequential and block synthesis, gene cloning and combinations of these techniques. The solid phase sequential procedure can be performed using established automated methods such as by use of an automated peptide synthesizer. In this procedure an α-amino protected amino acid is bound to a resin support. The resin support employed can be any suitable

5

resin conventionally employed in the art for the solid phase preparation of polypeptides, preferably polystyrene which has been cross-linked with from 0.5 to about 3 percent divinyl benzene, which has been either chloromethylated or hydroxymethylated to provide sites for ester formation with the initially introduced α-amino protected amino acid.

An example of a hydroxymethyl resin is described by Bodanszky et al., Chem. Ind. (London) 38, 1597-98 (1966). A chloromethylated resin is completely available from Bio Rad Laboratories, Richmond, California, and the preparation of such a resin is described by Stewart et al., "Solid Phase Peptide Synthesis" (Freeman & Co., San Francisco 1969), Chapter 1, pp. 1-6. The protected amino acid can be bound to the resin by the procedure of Gisin, Helv. Chem Acta, 56, 1476 (1973). Many resin bound, protected amino acids are commercially available. As an example, to prepare a polypeptide of this invention wherein the carboxy terminal end is a Thr residue, a tert-butyloxycarbonyl (Boc) protected Thr bound to a benzylated, hydroxymethylated phenylacetamidomethyl (PAM) resin can be used and is commercially available.

Following the coupling of the α-amino protected amino acid to the resin support, the protecting group is removed using any suitable procedure such as by using trifluoroacetic acid in methylene chloride, trifluoroacetic acid alone, or HCl in dioxane. The deprotection is carried out at a temperature between $0°C$ and room temperature. Other standard cleaving reagents and conditions for removal of specific α-amino protecting groups may be used. After removal of the α-amino protecting group the other amino protected amino acids are coupled step-wise in the desired order. Alternatively, multiple amino acid groups may be coupled by the solution method prior to coupling with the resin supported amino acid sequence.

The α-amino protecting group employed with each amino acid introduced into the polypeptide sequence may be any such protecting group known to the art. Among the classes of α-amino protecting groups contemplated are (1) acyl type protecting groups such as: formyl, trifluoroacetyl, phthalyl, toluenesulfonyl (tosyl), benzenesulfonyl, nitrophenylsulfenyl, tritylsulfenyl, o-nitrophenoxyacetyl and y-chlorobutyryl; (2) aromatic urethan type protecting groups such as benzyloxycarbonyl and substituted benzyloxycarbonyl such as p-chlorobenzyloxycarbonyl, p-nitrobenzyloxycarbonyl, p-bromobenzyloxycarbonyl, p-methoxybenzyloxycarbonyl, 1-(p-biphenylyl)-1-methylethoxycarbonyl, α,α-dimethyl-3,5-dimethoxybenzyloxycarbonyl and benzhydryloxycarbonyl; (3) aliphatic urethan protecting groups such as tert-butyloxycarbonyl (Boc), diisopropylmethoxycarbonyl, isopropyloxycarbonyl, ethoxycarbonyl and allyloxycarbonyl; (4) cycloalkyl urethan type protecting groups such as cyclopentyloxycarbonyl, adamantyloxycarbonyl and cyclohexyloxycarbonyl; (5) thiourethan type protecting groups such as phenylthiocarbonyl; (6) alkyl type protecting groups such as triphenylmethyl (trityl) and benzyl; (7) trialkylsilane groups such as trimethylsilane. The preferred α-amino protecting group is tert-butyloxycarbonyl.

The selection of an appropriate coupling reagent is within the skill of the art. A particularly suitable coupling reagent where the amino acid to be added is Gln, Asp or Arg is N,N'-diisopropylcarbodiimide and 1-hydroxybenzotriazole. The use of these reagents prevents nitrile and lactam formation. Other coupling agents are (1) carbodiimides (e.g., N,N'-dicyclohexylcarbodiimide and N-ethyl-N'-(y-dimethylaminopropylcarbodiimide)); (2) cyanamides (e.g., N,N-dibenzylcyanamide); (3) ketenimines; (4) isoxazolium salts (e.g., N-ethyl-5-phenyl isoxazolium-3'-sulfonate); (5) monocyclic nitrogen containing heterocyclic amides of aromatic character containing one through four nitrogens in the ring such as imidazolides, pyrazolides and 1,2,4-triazolides. Specific heterocyclic amides that are useful include N,N'-carbonyldiimidazole and N,N'-carbonyl-di-1,2,4-triazole; (6) alkoxylated acetylene (e.g., ethoxyacetylene); (7) reagents which form a mixed anhydride with the carboxyl moiety of the amino acid (e.g., ethylchloroformate and isobutylchloroformate) or the symmetrical anhydride of the amino acid to be coupled (e.g., Boc-Arg-O-Arg-Boc) and (8) nitrogen containing heterocylic compounds having a hydroxy group on one ring nitrogen (e.g., N-hydroxyphthalimide, N-hydroxysuccinimide and 1-hydroxybenzotriazole). Other activating reagents and their use in peptide coupling are described by Kapoor, J. Pharm. Sci., 59, pp 1-27 (1970). Applicants prefer the use of the symmetrical anhydride as a coupling reagent.

Each protected amino acid or amino acid sequence is introduced into the solid phase reactor in about a fourfold excess and the coupling is carried out in a medium of dimethylformamide: methylene chloride (1:1) or in dimethylformamide alone or preferably methylene chloride alone. In cases where incomplete coupling occurs, the coupling procedure is repeated before removal of the α-amino protecting group, prior to the coupling of the next amino acid in the solid phase reactor. The success of the coupling reaction at each stage of the synthesis is monitored by the ninhydrin reaction as described by E. Kaiser et al, Analyt. Biochem. 34, 595 (1970).

After the desired amino acid sequence has been obtained, the peptide is removed from the resin. This can be done by hydrolysis such as by treatment of the resin bound polypeptide with a solution of dimethyl sulfide, p-cresol and thiocresol in dilute aqueous hydrofluoric acid.

As is known in the art of solid phase peptide synthesis many of the amino acids bear functionalities requiring protection during the chain preparation. The use and selection of the appropriate protecting group is within the ability of those skilled in the art and will depend upon the amino acid to be protected and the presence of other protected amino acid residues on the peptide. The selection of such a side chain protecting group is critical in that it must be one which is not removed by cleavage during cleavage of the protecting group of the α-amino moiety. For example, suitable side chain protecting groups for lysine are benzyloxycarbonyl and substituted benzyloxycarbonyl said substituent being selected from halo (e.g., chloro, bromo, fluoro) and nitro (e.g., 2-chlorobenzyloxycarbonyl, p-nitrobenzyloxycarbonyl, 3,4- dichlorobenzyloxycarbonyl), tosyl, t-amyloxycarbonyl, t-butyloxycarbonyl and diisopropylmethoxycarbonyl. The alcoholic hydroxyl group of threonine and serine can be protected with an acetyl, benzoyl, tert-butyl, trityl, benzyl, 2,6-dichlorobenzyl or benzyloxycarbonyl group. The preferred protecting group is benzyl.

These groups are removed by procedures well known in the art. Typically protecting group removal is done after the peptide chain synthesis is complete but the protecting groups can be removed at any other appropriate time.

The peptides of this invention are immunosuppressant agents and can be useful in treating any disease or condition where suppression of humoral and/or cell mediated immunity is desirable such as in the treatment of rheumatoid arthritis and to prevent post kidney, liver or heart transplantation rejection. The peptides of this invention produce immunosuppression primarily by their ability to suppress proliferation of lymphocytes. The term "patient" as used herein is taken to mean mammals such as primates, including humans, sheep, horses, bovine cows and bulls, pigs, dogs, cats, rats and mice.

Although some of the peptides of this invention may survive passage through the gut following oral administration, parenteral administration is preferred, for example, subcutaneous, intravenous, intramuscular, or intraperitoneal administration; by depot injection or by implant preparation.

For parenteral administration the compounds may be administered as injectable dosages of a solution or suspension of the compound in a physiologically acceptable diluent with a pharmaceutical carrier which can be a sterile liquid such as water or oils with or without the addition of a surfactant and other pharmaceutically acceptable adjuvants. Illustrative of oils which can be employed in these preparations are those of petroleum, animal, vegetable, or synthetic origin, for example, peanut oil, soybean oil, and mineral oil. In general, water, saline, aqueous dextrose and related sugar solutions, ethanol and glycols such as propylene glycol or polyethylene glycol are preferred liquid carriers, particularly for injectable solutions.

The compounds can be administered in the form of a depot injection or implant preparation which may be formulated in such a manner as to permit a sustained release of the active ingredient. The active ingredient can be compressed into pellets or small cylinders and implanted subcutaneously or intramuscularly as depot injections or implants. Implants may employ inert materials such as biodegradable polymers or synthetic silicones, for example, Silastic, silicone rubber manufactured by the Dow-Corning Corporation.

In order to maximize the effect of the peptides of this invention it is preferable to employ a macromolecular carrier such as serum albumin, i.e., human serum albumin, where the patient is a human. Other carriers are, for example, keyhole limpet hemocyanin or a synthetic polymer such as poly(D-Glu, D-Lys). Attachment of the peptide to the carrier can be by one of several methods, including linking through a peptide Lys using glutaraldehyde (Reichlin, Methods Enzymol, 70: 159-165, 1980) or DCC procedures ( for example, Atassi et al., Biochem. Biophys Acta 670 300-302,1981), through a peptide Asp or Glu using DCC (Bauminger et al., Methods Enzymol, 70, 151-159, 1980), through a peptide Tyr using bis-diazotized benzidine (Walter, et al., Proc. Nat. Acad. Sci. USA 77: 5197-5200, 1980), through photochemical attachment sites (Parker et al., Cold Spring Harbor Symposium-Modern Approaches to Vaccines, Ed. Chanock & Lerner, Cold Spring Harbor Press, New York, 1983, in press), or through a peptide Cys (Liu et al., Biochem, 18: 690-697, 1979). Peptide carrier conjugates are separated from excess free peptide by dialysis or gel filtration. The level of loading of the peptide on the carrier can be determined either using a radioactive tracer to establish the loading level in a particular procedure, by HPLC of uncomplexed vs. complexed peptide, or by quantitative amino acid analysis of the conjugate, in comparison with the unloaded carrier. It is convenient, when using the latter technique, to incorporate a unique non-natural amino acid into the peptide, at the N-terminal or C-terminal side, such as Nle, which can then serve as a quantitative marker for peptide incorporation, as measured by amino acid analysis of the conjugate. This Nle can also function as a spacer between the immunosuppresive structure and any amino acid incorporated to facilitate attachment, such as Cys, Lys, or Tyr, as described above.

The peptides of this invention are immunosuppresant agents and suppress the lymphocyte system of patients by suppressing lymphocyte proliferation. While the peptides of this invention suppress proliferation of both T- and B-Cells, a particular peptide may preferentially suppress either T-lymphocytes or B-lymphocytes. However, all the peptides of this invention will suppress T-lymphocyte proliferation.

The quantity of peptide administered will vary depending on the patient, mode of administration, severity of the disease or condition and can be any effective amount. Repetitive daily administration of the peptides is desirable. The immunosuppressively effective amount of a peptide of the invention and the amount effective to suppress the lymphocyte system and lymphocyte proliferation can be from about 0.001 mg/kg to about 10 mg/kg of patient body weight per day. Preferably the peptide will be administered in unit dosage form from one to four doses per day of from, for example, 10 mg per dose.

## EXAMPLES

This invention is illustrated by the following nonlimiting examples.

## EXAMPLE 1

Preparation of H-Leu-Arg-Lys-Leu-Arg-Lys-Arg-Leu-Leu-Arg-Asp-Ala-Asp-Asp-Leu-NH$_2$

The peptide was synthesized by solid-phase methods using 0.1 mmol of a 0.48 mmol/g p-methylbenzhydryl amine resin. Double symmetrical anhydride couplings were performed with 2.0 mmol N$^\alpha$-Boc-amino acid (Peptides International). The side chain protection utilized was: Asp(Chx), Arg(Tos), Lys(2-ClZ). Upon completion of the synthesis the N$^\alpha$-Boc protection was removed with 50% trifluoroacetic acid in methylene chloride. The resin was washed three times with methylene chloride, neutralized with three washings of 10% diisopropylethylamine in methylene chloride, washed three times with methylene chloride, and dried *in vacuo*. The peptide was deprotected and cleaved from the resin with HF containing 2% anisole at 0° C, for 35 min. The HF was removed *in vacuo* at 0°, the peptide precipitated with ethyl ether, extracted from the resin with 30% aqueous acetic acid and lyophilized.

The peptide was purified by desalting on a 92 x 2.6 cm Sephadex G-15 column in 5% aqueous acetic acid and lyophilized. Preparative HPLC was performed on a C$^{18}$ Vydac 218TP1010 (250 x 10 mm) column with 24% acetonitrile in 0.1% aqueous trifluoroacetic acid at 5 ml/min. The major peak was collected and lyophilized.

TLC: [(Merck 5715 20 x 20 cm Silica gel 60 plates (0.25 mm thickness))] n-butanol/acetic acid/water/pyridine (6:1.2:4.8:6) (TLC I) Rf = 0.52; FAB-MS: (M + H) = 1879 ± 1 m.u. (calcd. 1879.2). Amino acid analysis: (6N HCl hydrolysis; 24 hr at 106° C). Asx 3.06 (3); Ala 0.99 (1); Leu 4.97 (5); Lys 2.01 (2); Arg 4.01 (4). 72% peptide content by weight.

## EXAMPLES 2-4

In substantially the same manner as indicated in Example 1, the following peptides were prepared.

| Example No. | Peptide |
|---|---|
| 2 | H-Thr-Arg-Leu-Thr-Arg-Lys-Arg-Gly-Leu-Lys-Leu-Ala-Thr-Ala-Leu-NH$_2$ |
| 3 | H-Leu-Gln-Asn-Arg-Arg-Gly-Leu-Asp-Leu-Leu-Phe-Leu-Lys-Glu-Gly-Gly-Leu-NH$_2$ |
| 4 | H-Glu-Val-Val-Leu-Gln-Asn-Arg-Arg-Gly-Leu-Leu-OH |

The peptides of Examples 2-4 have the following physical properties and amino acid analysis.

| Example No. | Molecular Weight | | HPLC tr(min) (15-40% gradient) |
|---|---|---|---|
| | Theoretical | FAB-MS(M + H) ±1 mu | |
| 2 | 1695 | 1696 | 9.8 |
| 3 | 1940 | 1940 | 17.6 |
| 4 | 1295 | 1297 | 10.8 |

| Amino Acid Analysis* | | | | | | | | | | |
|---|---|---|---|---|---|---|---|---|---|---|
| Example No. | Asx | Thr | Glx | Gly | Ala | Val | Leu | Phe | Lys | Arg |
| 2 | | 3.01(3) | | 1.07(1) | 2.05(2) | | 3.94(4) | | 2.17(2) | 2.20(3) |
| 3 | 2.01(2) | | 2.03(2) | 3.10(3) | | | 6.06(6) | 1.00(1) | 0.99(1) | 1/81(2) |
| 4 | 1.03(1) | | 2.05(2) | 1.05(1) | | 0.95(2) | 2.92(3) | | | 2.00(2) |

* 6N HCl, 20 hours at 106°C

## EXAMPLE 5

### Preparation of the BSA conjugate of H-Thr-Arg-Leu-Thr-Arg-Lys-Arg-Gly-Leu-Lys-Leu-Ala-Thr-Ala-Leu-NH₂

The peptide was coupled to Bovine Serum Albumin (BSA) by reaction with 1-ethyl-3(3-dimethylaminopropyl)carbodiimide hydrochloride (EDAC). The peptide (1mM) in 1.84 ml of $H_2O$ was reacted (15-30 min.) with 0.16 ml of BSA (25 mg/ml) previously treated with 40 mg EDAC for 1-30 minutes in $H_2O$. The activated BSA sample was added dropwise with stirring to the peptide solution and then incubated 10 hours at room temperature. Then 2.0 ml of 1.0 M glycine were added and the solution was rotated overnight at 4°C. To remove excess reagent and nonconjugated peptide, samples were dialyzed extensively at 4°C first against 15 x 4L changes of distilled water, then against 2 x 4 L changes of CELLGRO (Mediatech) tissue culture media. Samples were sterile-filtered through a 0.45 μm cellulose acetate membrane just prior to their addition to cell cultures. Extent of peptide coupling was determined by reverse-phase HPLC.

The BSA conjugates of the peptides 1, 3 and 4 were prepared by first activating the peptide with EDAC in the same manner as above and then reacting with BSA.

## EXAMPLE 6

### Inhibition of ³H-Thymidine Incorporation in Mixed Lymphocyte Cultures by Various Peptides

Spleens were aseptically removed and teased apart in RPMI 1640 culture media to obtain single cell suspensions. Erythrocytes were lysed by suspending cells in ACK buffer (0.155 M $NH_4Cl$, 0.1 mM EDTA, 0.01 M $KHCO_3$) and the remaining leukocytes washed. C57BL/6 ($5 \times 10^5$ cells) and DBA/2 ($5 \times 10^5$ cells) leukocytes were co-cultured in 0.1 ml RPMI-1640, supplemented with 2% fetal calf serum (FCS), in 96-well

9

microtiter plates. Various concentrations of peptides were added at the initiation of cell culture. Following a 120 hour incubation (37° C), including a pulse with 1 $\mu$Ci of [$^3$H]TdR in the final 18 hours of culture, the cells were harvested with an automated cell harvester unit onto filter paper strips and placed in ACS scintillation fluid (Amersham, Oakville, Ontario). The amount of [$^3$H]TdR incorporation was then measured in a Beckman LS 7800 liquid scintillation counter.

| Compound | [Peptide][b] $\mu$M | $^3$H-Thymidine Incorporation (cpm) | % Inhibition |
|---|---|---|---|
| BSA control[a] | none none | 6621 ± 442 8023 ± 444 | -- -- |
| Example 3 | 24 12 | 1328 ± 421 3417 ± 263 | 80 57 |
| Example 1 | 18 9 | 1800 ± 412 3267 ± 457 | 73 59 |
| Example 2 | 28 14 | 2209 ± 1471 4406 ± 848 | 67 45 |
| Example 4 | 8 4 | 3063 ± 372 5751 ± 407 | 54 28 |

[a] BSA alone conjugated with 1-ethyl-3(3-dimethylaminopropyl)-carbodiimide-HCl
[b] Concentration of BSA-conjugated peptide

## EXAMPLE 7

Inhibition of $^3$H-Thymidine Incorporation in Anti-T$_3$ Stimulated Lymphocytes by Various Peptides

Human peripheral blood mononuclear leukocytes MNL were isolated by Ficoll-Hypaque density sedimentation. MNL (105) were cultured with anti-T$_3$ (1:10,000 dilution; Ortho Diagnostics) in 0.1 ml DMEM, supplemented with 2% FCS, in 96-well microtiter plates. Following a 72 hour incubation (37° C), including a pulse with 1 $\mu$Ci of [$^3$H]TdR in the final 18 hours of culture, the cells were harvested and counted as described in Example 6.

| Compound | [Peptide][b] μM | ³H-Thymidine Incorporation (cpm) | % Inhibition |
|---|---|---|---|
| BSA control[a] | none none | 43934 ± 5076 57388 ± 6153 | -- -- |
| Example 3 | 24 12 | 14027 ± 867 31667.±.4729 | 68 45 |
| Example 1 | 18 9 | 21984 ± 3824 38521 ± 5349 | 50 33 |
| Example 2 | 28 14 | 20041 ± 3331 44151 ± 2151 | 54 23 |
| Example 4 | 8 4 | 39248 ± 5251 54331 ± 4717 | 11 5 |

[a]      BSA      alone      conjugated      with 1-ethyl-3(3-dimethylaminopropyl)-carbodiimide-HCl
[b] Concentration of BSA-conjugated peptide

## Claims

1. A peptide of the formula

Tn-A-B-C-D-Tc

wherein

Tn is an amino terminal residue selected from hydrogen, one or two alkyl groups of from 1 to 6 carbon atoms, one or two acyl groups of from 2 to 10 carbon atoms, carbobenzyloxy, and t-butyloxycarbonyl;

A is a peptide fragment containing from 3 to 12 residues of any amino acid;

B is a peptide fragment containing 2 or 3 amino acid residues selected from Arg and Lys;

C is a peptide fragment containing 2 or 3 amino acid residues selected from Gly, Leu, Ile, and Thr;

D is a peptide fragment containing from 1 to 12 residues of any amino acid; and

Tc is a carboxy terminal residue selected from OH, $(C_1-C_6)$alkoxy, amino, mono- or di-$(C_1-C_4)$alkyl substituted amino, and benzylamino.

2. A peptide of claim 1 wherein C is a peptide fragment containing 2 or 3 amino acid residues selected from Gly and Leu.

3. A peptide of claim 1 or 2 wherein Tn is hydrogen.

4. A peptide of claim 1 or 2 wherein Tc is selected from OH and amino.

5. A peptide of claim 1 or 2 wherein A is a peptide fragment containing from 3 to 6 residues of any natural amino acid.

6. A peptide of claim 1 or 2 wherein D is a peptide fragment containing from 1 to 12 residues of any natural amino acid.

7. A peptide of claim 1 which is H-Leu-Arg-Lys-Leu--Arg-Lys-Arg-Leu-Leu-Arg-Asp-Ala-Asp-Asp-Leu-NH₂.

8. A peptide of claim 1 which is H-Thr-Arg-Leu-Thr--Arg-Lys-Arg-Gly-Leu-Lys-Leu-Ala-Thr-Ala-Leu-NH₂.

9. A peptide of claim 1 which is H-Leu-Gln-Asn-Arg--Arg-Gly-Leu-Asp-Leu-Leu-Phe-Leu-Lys-Glu-Gly-Gly-Leu-NH₂.

10. A peptide of claim 1 which is H-Glu-Val-Val-Leu--Gln-Asn-Arg-Arg-Gly-Leu-Leu-OH.

11. A compound according to any one of claims 1 to 10 for use as a medicine.

12. Use of a compound of anyone of claims 1 to 10 for preparing a medicament for modulating or suppressing the lymphocyte system.

13. Use of a compound of anyone of claims 1 to 10 for preparing a medicament for modulating or suppressing the lymphocyte proliferation.

11

14. A pharmaceutical composition comprising a compound of anyone of claims 1 to 10 in admixture with a pharmaceutically acceptable carrier.

15. A process for preparing a compound of anyone of claims 1 to 10 by solid phase sequential and block synthesis. gene cloning or a combination thereof.

16. A solid phase sequential and block synthesis process for preparing a compound of any one of claims 1 to 10 which comprises binding a suitably protected amino acid corresponding to the carboxy terminal amino acid of the A group to an activated resin support and subsequently binding to other alpha amino protected amino acids of the A, B, C and D groups in order from the carboxy to amino terminal end to the terminal amino group of the growing peptidic chain which has meanwhile been exposed by removing its amino protecting group.

Claims for the following Contracting States: ES,GR

1. A process for preparing a peptide of the formula

Tn-A-B-C-D-Tc

wherein

Tn is an amino terminal residue selected from hydrogen, one or two alkyl groups of from 1 to 6 carbon atoms, one or two acyl groups of from 2 to 10 carbon atoms, carbobenzyloxy, and $\underline{t}$-butyloxycarbonyl;

A is a peptide fragment containing from 3 to 12 residues of any amino acid;

B is a peptide fragment containing 2 or 3 amino acid residues selected from Arg and Lys;

C is a peptide fragment containing 2 or 3 amino acid residues selected from Gly, Leu, Ile, and Thr;

D is a peptide fragment containing from 1 to 12 residues of any amino acid; and

Tc is a carboxy terminal residue selected from OH, $(C_1-C_6)$alkoxy, amino, mono- or di-$(C_1-C_4)$alkyl substituted amino. and benzylamino

and the pharmaceutically acceptable salts thereof by solid phase sequential and block synthesis. gene cloning, or combinations thereof.

2. A solid phase sequential and block synthesis process for preparing a peptide derivative of the formula

Tn-A-B-C-D-Tc

wherein

Tn is an amino terminal residue selected from hydrogen, one or two alkyl groups of from 1 to 6 carbon atoms, one or two acyl groups of from 2 to 10 carbon atoms, carbobenzyloxy, and $\underline{t}$-butyloxycarbonyl;

A is a peptide fragment containing from 3 to 12 residues of any amino acid;

B is a peptide fragment containing 2 or 3 amino acid residues selected from Arg and Lys;

C is a peptide fragment containing 2 or 3 amino acid residues selected from Gly, Leu, Ile, and Thr;

D is a peptide fragment containing from 1 to 12 residues of any amino acid; and

Tc is a carboxy terminal residue selected from OH, $(C_1-C_6)$alkoxy, amino, mono- or di-$(C_1-C_4)$alkyl substituted amino, and benzylamino

and the pharmaceutically acceptable salts thereof which comprises binding a suitably protected amino acid corresponding to the carboxy terminal amino acid of the A group to an activated resin support and subsequently binding the other alpha amino protected amino acids of the A, B, C, and D groups in order from the carboxy to amino terminal end to the terminal amino group of the growing peptidic chain which has meanwhile been exposed by removing its amino protecting group.

3. A process according to claims 1 or 2 for preparing a compound wherein C is a peptide fragment containing 2 or 3 amino acid residues selected from Gly and Leu.

4. A process according to claims 1 or 2 for preparing a compound wherein Tn is hydrogen.

5. A process according to claims 1 or 2 for preparing a compound wherein Tc is selected from OH and amino.

6. A process according to claims 1 or 2 for preparing a compound wherein A is a peptide fragment containing from 3 to 6 residues of any natural amino acid.

7. A process according to claims 1 or 2 for preparing a compound wherein D is a peptide fragment containing from 1 to 12 residues of any natural amino acid.

8. A process according to claims 1 or 2 for preparing a compound which is H-Leu-Arg-Lys-Leu-Arg-Lys-Arg-Leu-Leu-Arg-Asp-Ala-Asp-Asp-Leu-$NH_2$.

9. A process according to claims 1 or 2 for preparing a compound which is H-Thr-Arg-Leu-Thr-Arg-Lys-Arg-Gly-Leu-Lys-Leu-Ala-Thr-Ala-Leu-$NH_2$.

10. A process according to claims 1 or 2 for preparing a compound which is H-Leu-Gln-Asn-Arg-Arg-Gly-Leu-Asp-Leu-Leu-Phe-Leu-Lys-Glu-Gly-Gly-Leu-$NH_2$.

11. A process according to claims 1 or 2 for preparing a compound which is H-Glu-Val-Val-Leu-Gln-Asn-Arg-Arg-Gly-Leu-Leu-OH.

12. Use of a compound of claim 1 or 2 for preparing a medicament for modulating or suppressing the lymphocyte system.

13. Use of a compound of claim 1 or 2 for preparing a medicament for modulating or suppressing the lymphocyte proliferation.